# EUROPEAN PATENT SPECIFICATION

(11) **EP 3 322 373 B1**
(45) Date of publication and mention of the grant of the patent: **14.04.2021**
(21) Application number: 16730136.5
(22) Date of filing: 01.06.2016
(51) Int. Cl.: A61B 90/30, A61F 5/00

(54) **LIGHTED GASTRIC MEDICAL DEVICE ASSEMBLY**
BELEUCHTETE GASTRISCHE MEDIZINISCHE VORRICHTUNGSANORDNUNG
ENSEMBLE DISPOSITIF MÉDICAL GASTRIQUE ÉCLAIRÉ

(30) Priority: 15.07.2015 US 201562192728 P
(43) Date of publication of application: 23.05.2018
(73) Proprietor: Cook Medical Technologies LLC, Bloomington, IN 47402 (US)
(72) Inventor: FENG, Brian, Bloomington, IN 47403 (US); MELSHEIMER, Jeff, Springville, IN 47462 (US); KAMEL, Amro, Bloomington, IN 47402 (US); EELLS, Robert, Ambler, PA 19002 (US); SHERWINTER, Danny, New York, NY 11210 (US)
(74) Representative: Mathys & Squire
(86) International application number: PCT/US2016/035215
(87) International publication number: WO 2017/011085

(56) References cited:
- EP-A1- 2 870 919
- WO-A1-2012/141679
- WO-A1-2014/043269
- WO-A1-2015/069506
- US-A1- 2004 092 892
- US-A1- 2005 251 158

## Description

### Technical Field

The present disclosure relates generally to gastric medical device assemblies for performing a medical procedure, and more particularly to a lighted assembly that includes a gastric bougie slidably received on a gastric catheter.

### Background

Sleeve gastrectomy is a surgical weight loss procedure in which the stomach is reduced to about 25% of its original size by surgical removal of a large portion of the stomach along the greater curvature. The result leaves the stomach with a generally sleeve tube like shape. The procedure is generally performed laparoscopically. Sleeve gastrectomy typically involves sliding a gastric bougie through the mouth of the patient for positioning in the stomach. The procedure may include collapsing the stomach around the gastric bougie in order to guide the surgeon in resecting the stomach adjacent the gastric bougie and stapling along the incision utilizing laparoscopic tools and vision.

Although the gastric bougie can be effective in assisting the physician in better visualizing an incision path and potential staple line, it is sometimes difficult to properly position the bougie and confirm that proper positioning visually. Misplacement of the bougie can sometimes result in the stapler running over the tip or side of the bougie. It is also difficult to ensure that the tip of the bougie is properly positioned to determine where the gastric sleeve should begin, and it is sometimes difficult to maneuver the bougie against the lessor curvature of the stomach. The present disclosure is directed toward one or more of the problems set forth above.

WO 2012/141679 A1 relates to a bougie (e.g. a sizing tube) that is introduced prior to performing laparoscopic sleeve gastrectomy, in an orogastric fashion which enables a surgeon to accurately size a remaining portion of a patient's stomach and/or remove an excess portion dissected from the stomach. The bougie takes the form of a clear tube, and comprises markings which indicate one or more sets of standardized units to generally determine a more precise amount of stomach tissue to maintain. Moreover, the tube is hollow, thus enabling an endoscopic camera and/or a light to be inserted, for example, while the tube is being introduced into the patient or alternatively, at a later time. The tube comprises a camera and/or a built in light. US 2004/0092892 describes an apparatus and method for treatment of morbid obesity. The device includes an artificial stoma device, a combined gastrointestinal sleeve device and permanent detachable attachment systems, and devices for deploying the components of the system. WO 2015/069506 describes a gastrectomy device including an elongated member and a tube. The elongated member has a proximal end and a distal end and defines a longitudinal side window disposed adjacent the distal end. The elongated member defines a first longitudinal channel, a second longitudinal channel, and a plurality of side apertures. The first longitudinal channel is in communication with the longitudinal side window and the plurality of side apertures is in communication with the second longitudinal channel. The tube extends through the first longitudinal channel. An array of lights is associated with the tube to provide illumination. The tube is movable through the elongated member between a first state, in which the tube is disposed within the first longitudinal channel of the elongated member, and a second state, in which a portion of the tube extends through the longitudinal side window of the elongated member.

The present invention is defined by the features of the independent claim. Preferred embodiments are given in the dependent claims.

In one aspect, a gastric medical device assembly includes a gastric bougie that defines at least one lumen. A gastric catheter has a distal segment extending distally from the gastric bougie, a middle segment slidably received in a first lumen of the at least one lumen, and a proximal segment extending proximally from the gastric bougie. A light emitter is attached to a distal portion of at least one of the gastric bougie and the gastric catheter, and has a luminosity sufficient to penetrate through, and be seen on an opposite side of, a stomach wall. At least one of the gastric bougie and gastric catheter define a plurality of side ports.

In another aspect, a method of performing a medical procedure with the gastric medical device assembly includes moving the gastric catheter into a stomach. The gastric bougie is slid over the gastric catheter and into the stomach. Material from the gastrointestinal tract is suctioned into the side ports of the gastric medical device assembly. A location of the light emitter is compared to a gastrointestinal tract landmark from an observation point outside of the stomach.

### Brief Description of the Drawings

Fig. 1 is a sectioned side schematic view of a gastric medical device assembly according to one aspect of the present disclosure;
Fig. 2 is a sectioned view through a gastric medical device assembly according to another embodiment of the present disclosure;
Fig. 3 is a sectioned view through a gastric medical device assembly according to another embodiment of the present disclosure;
Fig. 4 is a view of a gastric catheter according to the present disclosure;
Fig. 5 is a side view of a gastric medical device assembly according to another aspect of the present disclosure;
Fig. 6 is a side view of a gastric medical device assembly according to still another embodiment of the present disclosure;
Fig. 7 is a section view through the medical device assembly of Fig. 6 as viewed along section lines A-A;
Fig. 8 is a sectioned view through the gastric medical device assembly of Fig. 6 as viewed along section line B-B;
Fig. 9 is a sectioned view through the gastric medical device assembly of Fig. 6 as viewed along section line C-C;
Fig. 10 is a side schematic view of a gastric catheter according to the present disclosure being slid into the stomach of a patient;
Fig. 11 is a schematic view of the stomach after the gastric catheter has been properly positioned in the stomach;
Fig. 12 is a schematic view similar to Figs. 10 and 11 after the gastric bougie has been slid over the gastric catheter and into the stomach;
Fig. 13 is a side schematic view during suctioning and activation of lights to outline a staple line for a sleeve gastrectomy; and
Fig. 14 is a schematic view similar to Figs. 10-13 immediately after a portion of the stomach has been cut away and stapled in the performance of a sleeve gastrectomy utilizing the gastric medical device assembly of the present disclosure.

### Detailed Description

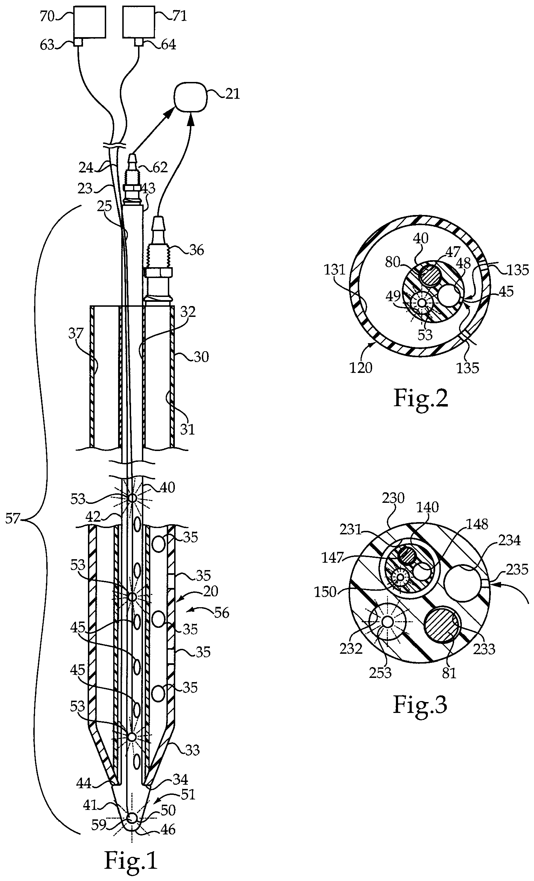

Referring initially to Fig. 1, a gastric medical device assembly 20 includes a gastric bougie 30 and a gastric catheter 40. In one specific example, gastric catheter 40 may have a diameter in the range of 18-20 French, and the gastric bougie may have a diameter in the range of 32-60 French. In any event, in all cases, the gastric medical device assembly 20 will include a matched set of a gastric catheter 40 and a gastric bougie 30, which may be used by a physician to perform a suitable medical procedure, such as a sleeve gastrectomy. In all cases the gastric catheter 40 will be longer than the gastric bougie such that a distal segment 41 of the gastric catheter will extend distally from the gastric bougie 30, a middle segment 42 will be slidably received in a lumen 32 of the gastric bougie 30, and a proximal segment 43 will extend proximally from the gastric bougie 30. A light emitter 50 is attached to a distal portion of at least one of the gastric bougie 30 and the gastric catheter 40, and has a luminosity sufficient to penetrate through, and be seen on an opposite side of, a stomach wall. In the embodiment of Fig. 1, light emitter 50 is attached to a distal portion 51 of the gastric catheter 40. Having a luminosity sufficient to be seen on an opposite side of the stomach wall will aid a physician in attempting to visually locate the medical device assembly 20 from outside the stomach when performing laparoscopic surgery. At least one of the gastric bougie 30 and the gastric catheter 40 includes side ports fluidly connected to appropriate lumens. For instance, a side port may be used to evacuate stomach contents, and/or facilitate collapse of the stomach around gastric bougie 30 to facilitate the sleeve gastrectomy. In the embodiment of Fig. 1, gastric catheter 40 includes a plurality of side ports 45 that are fluidly connected to a fitting 62, such as a female luer fitting, which may be connected to an appropriate external device, such as suction device 21 in a manner well known in the art. In addition, the Fig. 1 embodiment includes gastric bougie 30 having a plurality of side ports 35 fluidly connected to a lumen 31 that terminates at its proximal end with a fitting 36, which may also be connected to a suitable external device, such as suction device 21. In the Fig. 1 embodiment, gastric bougie 30 includes at least three lumens 31, 32 and 37, but a gastric bougie having any number of lumens from one to four or more would also fall within the intended scope of the present disclosure.

In the embodiment of Fig. 1, only the lumen 32 of gastric bougie 30 opens at the distal end, with the other lumens 31 and 37 being closed distally in the vicinity of tapered distal end 33. Gastric catheter 40 includes a distal stop surface 44. The gastric bougie includes a tapered distal end 33 that terminates at a contact surface 34. The distal stop surface 44 blocks the gastric bougie 30 from being advanced past a position where a contact surface 34 is in contact with the distal stop surface 44 of gastric catheter 40. The light emitter 50 mounted in gastric catheter 40 may be positioned just proximal to a closed atraumatic distal tip 46 such that gastric catheter 40 has no lumens that open at a distal end. Light emitter 50 may be an electrical light 59 that receives electrical energy from an external electrical power source 71 via an energy transmission cable 24, which may take the form of an electrical circuit 25. Nevertheless, those skilled in the art will appreciate that the electric circuit for electric light 59 could be completely contained with its own battery power source within an appropriate lumen of the gastric medical device 20. On the otherhand, the energy transmission cable 24 may terminate at its proximal end in a plug 64 to facilitate quick connection to external electrical power source 71, such as a power source located in the operating room where the gastric medical device assembly 20 is to be used. Nevertheless, light emitter 50 could be an emitter node of a fiber optic cable, or could even be a chemoluminescent light source of the type known in the art. Gastric catheter 40 may also include a plurality of light emitters 53 that are distributed in a pattern 56 along a length 57 of gastric catheter 40. For instance, the pattern 56 of light emitters 53 may extend along a length associated with a patient's stomach to guide a staple line during the performance of a sleeve gastrectomy. In the illustrated embodiment, three light emitters 53 are shown, but any number of two or more light emitters 53 would fall within the scope of the present disclosure. In the embodiment of Fig. 1, light emitters 53 are connected to an external light source 70 by a separate energy transmission cable 24 that may take the form of a fiber optic cable 23. Fiber optic cable 23 may terminate at its proximal end with a plug 63 for easy connection in the operating room to external light source 70. Those skilled in the art will appreciate that the embodiment of Fig. 1 is shown as including both fiber optic light emitters 53 and an electrical light 59 for light emitter 50 in order to illustrate the variability of a gastric medical device assembly 20 according to the present disclosure. In a practical application, all of the light emitters 50 and 53 may likely be of a similar type Light emitters 50 and 53 may be different colors, may be able to change colors, and may have the ability to blink individually or in unison. Also, both the gastric catheter 40 and the gastric bougie 30 of gastric medical device 20 may be constructed of a suitable translucent or transparent plastic material so that the lights and associated circuits can be housed in dedicated lumens and isolated from the patient. The light generated by light emitters 50, 53 can pass through intervening walls and surfaces of the respective gastric catheter 40 and gastric bougie 30 and still be seen laparoscopically from outside of a patient's stomach. It should also be noted in the embodiment of Fig. 1 that the light emitter 50 is located outside of the gastric bougie 30, but the other light emitters 53 are positioned inside gastric bougie 30. Thus, gastric bougie 30 may be considered to be translucent with respect to light emitters 53.

Referring now in addition to Fig. 2, a gastric medical device assembly 120 according to another embodiment of the present disclosure includes a gastric bougie 130 with a single lumen 131 that receives a gastric catheter 40 of the type shown in Fig. 1. Gastric bougie 130 also includes a plurality of side ports 135 that may be fluidly connected to a plurality of side ports 45 of gastric catheter 40, such as for aspiration of fluids via lumen 48. Thus, in this embodiment, only the gastric catheter 40 may be connected to a suitable suction device 21 as shown in Fig. 1. Alternatively, the gastric bougie 130 may have no side ports but still allow for fluid communication between the area outside of gastric bougie 30 and side ports 45 of gastric catheter 40 via a clearance between the outside diameter of gastric catheter 40 and the inner diameter of the lumen 131 where the gastric bougie 130 includes a distal taper. The embodiment of Fig. 2 is also of interest for showing a deformable mandrel 80 that is received in lumen 47 of gastric catheter 40. Mandrel 80 may be metallic and may allow the physician to re-shape gastric catheter 40 by bending mandrel 80 in order to deflect the gastric catheter 40 into something other than a straight configuration to better facilitate steering the device through a patient's anatomy. Fig. 2 is also noteworthy for showing the light emitters 53 completely enclosed within a third lumen 49 of gastric catheter 40 so that the light emitters 53 and their energy transmission cable 24 are completely isolated from any fluids that may come in contact with the external surface of gastric catheter 40 or into lumen 131 of gastric bougie 30. Nevertheless, the translucency of gastric catheter 40 and gastric bougie 130 permit light from light emitters 53 to penetrate all the way through a patient's tissue, such as to be seen from the outside of a patient's stomach during a sleeve gastrectomy.

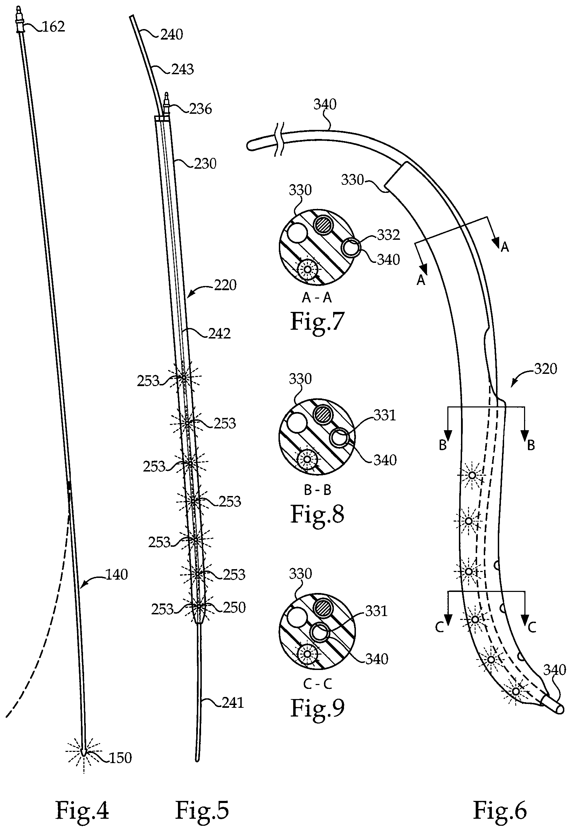

Referring now to Fig. 3, a sectioned view through an alternative gastric bougie 230 includes a first lumen 231 such as for receiving a gastric catheter according to the present disclosure therethrough. Gastric bougie 230 also differs from the earlier embodiment in that a separate lumen 232 houses the light emitters 253 and any associated energy transmission cable 24, whether the energy transmission be via a fiber optic cable or an electric circuit. Gastric bougie 230 also includes a deformable mandrel 81 received in a third lumen 233 in order to permit the physician to reshape gastric bougie 230 to better facilitate movement through the patient's anatomy, such as through a curved gastric tract. Finally, gastric bougie 230 may include a fourth lumen 234 that is fluidly connected toward the distal end to a plurality of side ports 235, and may be connected at its proximal end to a suitable fitting 236 for fluid connection to an external device, such as suction device 21 as shown in Fig. 1. The gastric bougie 230 of Fig. 3 may be utilized with a suitable gastric catheter according to the present disclosure, such as the gastric catheter 140 shown in Fig. 4 to comprise a gastric medical device assembly according to still another embodiment of this disclosure. Gastric catheter 140 differs from the gastric catheter 40 discussed earlier in that it includes only a single light emitter 150 positioned at its distal end. Gastric catheter 140 is also noteworthy for showing the ability to be reshaped, such as by the inclusion of an appropriate mandrel 147 therein, to provide steering and tip deflection as shown with the dashed lines. Finally, gastric catheter 140 may include a suitable fitting 162 at its proximal end for fluid connection to an internal lumen 148 of gastric catheter 140, which may or may not include its own set of side ports.

Fig. 5 shows a gastric medical device 220 utilizing the gastric bougie 230 of Fig. 3 in conjunction with a gastric catheter 240 that includes no light emitters. Nevertheless, like the earlier embodiment, gastric catheter 240 includes a distal segment 241 that extends distally from gastric bougie 230, a middle segment 242 positioned inside gastric bougie 230, and a proximal segment 243 extending proximally from gastric bougie 230. Instead of including a light emitter on the gastric catheter, this embodiment includes a first light emitter 250 positioned in the distal portion of gastric bougie 230, and a plurality of light emitters 253 extending in a pattern along the length of gastric bougie 230 positioned in a separate lumen 232 as shown in Fig. 3. For instance, this embodiment may be utilized by first positioning a gastric catheter 240 in the patient and then utilizing the lighter emitters 250, 253 of gastric bougie 230 in order to properly position gastric bougie 230 within the patient via laparoscopic viewing from outside the patient's stomach.

Referring now to Figs. 6-9, an alternative gastric medical device assembly 320 may include any combination of the features discussed earlier as far as side ports and lighting on one or both of the gastric catheter 340 and gastric bougie 330. However, this embodiment differs in that gastric bougie has rapid exchange features such that it includes an external groove 332 that transitions into an internal lumen 331 so that the gastric catheter 330 need not be too significantly longer than the gastric bougie 330. The section views of Fig. 7, Fig. 8 and Fig. 9 show briefly how the external groove 332 transitions into an internal lumen 331 for slidably receiving gastric catheter 340.

In review, a gastric catheter according to the present disclosure may be in the range of 18-20 French in diameter and may or may not contain a light emitter at its distal end. In addition, the gastric catheter may or may not include additional light emitters along a portion of its length, such as a portion of length that resides in the patient's stomach. The light emitters are preferably located inside the external surface of the respective device so that the external surfaces of the gastric bougie and catheter can be made smooth, and the lighting hardware is completely isolated from contact with patient tissue and fluids. The gastric catheter according to the present disclosure may include one or more lumens, where one of the lumens may be communicating with a plurality of side ports along a distal portion to facilitate aspiration of fluids from a patient's body cavity, such as a patient's stomach. According to the invention, the gastric catheter 40 includes an enlarged diameter feature near its distal end to act as a hard stop to keep the overlying gastric bougie 30 from being slid past contact with the distal stop surface 44, as shown in the embodiments of Fig. 1 and Fig. 4.

Also in review, a gastric bougie according to the present disclosure may have a diameter in the range of 32-60 French. The gastric bougie has one or more lumens with one of the lumens opening through a tapered distal end for receiving the gastric catheter therethrough. The gastric bougie of the present disclosure may or may not be equipped with side ports along a portion of the gastric bougie that might reside in a patient's stomach. If the gastric bougie does include side ports, they may communicate with a second lumen along its length to facilitate either fluid connection at a proximal end via a separate fitting or fluid connect directly with the side ports of the internal gastric catheter. The gastric bougie may include quick exchange features such as those shown in Figs. 6-9 which may start as a slit and transform into a groove which in turn transforms into an internal lumen so that the gastric bougie can be side loaded onto an underlying gastric catheter, thereby reducing the length necessary for the gastric catheter. Alternatively, the gastric bougie may have a larger side port at a location along its length which is in communication with a gastric catheter lumen so that the gastric bougie can be placed over an underlying gastric catheter using a known rapid exchange technique, again thereby reducing the length necessary for the gastric catheter.

One or both of the gastric catheter and gastric bougie may contain a fiber optic and/or an electric, and/or a chemoluminescent lighting system. Finally, these lighting systems may include proximal plugs to better ease attachment to existing light or power sources already available in an operating room. The gastric catheter and/or the gastric bougie may be equipped with a steerable feature, such as by positioning a suitable deformable mandrel within the appropriate device. Either the gastric catheter and/or the gastric bougie may also include radiopaque features to facilitate fluoroscopically guided visualization. Furthermore, one or both of the gastric catheter and gastric bougie may include echogenic features to facilitate ultrasonic guided visualization.

One strategy for making the gastric catheter may include cutting a length of medical grade tubing that has multiple lumens running along its entire length. The gastric catheter material may be transparent or translucent. One or more side ports may be formed in the "stomach" section of the gastric catheter and be made to communicate with only one of the multiple lumens. In a parallel process, a wiring harness may be fabricated, which has a light or lights near its distal end, and may include a switch and/or plug near its proximal end. A low profile lithium polymer battery may be connected into the electrical circuit in the case of electrical lights, or an external power source of the type described with regard to the embodiment of Fig. 1 may be utilized. The wiring harness is passed through a second lumen which includes a closed distal end. The distal end of the gastric catheter may be reformed using known techniques to close the light containing lumen and seal the ends of the other lumens to form a closed atraumatic distal tip 46 as shown in Fig. 1. The proximal end of the medical grade tubing may then be trimmed to length, and a female lure fitting 62, 162 may be attached to the lumen fluidly connected to the side ports. If a steering feature is included, a suitable steering mechanism can be incorporated into a third lumen, such as by including a mandrel 80 in a separate lumen 47 as shown in the embodiment of Fig. 2.

To make the gastric bougie, a length of medical grade tubing may be acquired which has one or more lumens running along its entire length. The material may be transparent or translucent to light emitters positioned therein. A plurality of side ports may be formed in the "stomach" section of the gastric bougie and communicate with one of the lumens. If a steerable feature is included, a mandrel may be inserted into a second lumen. Heat and pressure may be used to form an atraumatic tapered distal tip on the distal end of the tubing. The proximal end may then be trimmed to length, and if utilized, a female lure fitting may be attached to the lumen fluidly connected to the side ports, if any. If the gastric bougie itself will be illuminated, a separate wiring harness and/or fiber optic cable can be incorporated into a third lumen at this time. If a steering feature is desired, such as in the embodiment shown in Fig. 3, a suitable mandrel 81 may be incorporated into a fourth lumen at that time.

### Industrial Applicability

The present disclosure finds potential application in different medical procedures, including but not limited to sleeve gasterectomies and maybe gastric bypasses. The gastric medical device assembly of the present disclosure might also find potential application in other procedures, including but not limited to esophageal resections, anti-reflux surgery, bowel/colon resection, and maybe even hysterectomies.

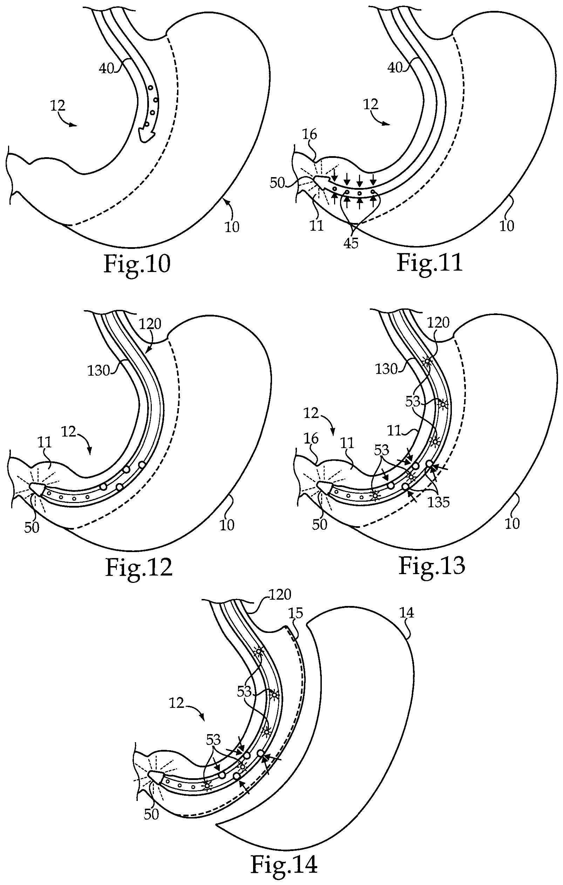

Referring now in addition to Figs. 10-14, a storyboard of events for performing a sleeve gastrectomy utilizing a gastric medical device assembly 120 according to the present disclosure is illustrated. The procedure may be initialized by using a standard trans-esophageal methodology by sliding a gastric catheter 40 into the gastric tract 12 of the patient. This process may be facilitated by utilizing a steerable feature as discussed earlier. As the gastric catheter 40 passes through the patient's stomach 10, laparoscopic visualization may be utilized in order to position the light emitter 50 at the distal end of gastric catheter 40 immediately adjacent the pylorus 16, and the body of the gastric catheter 40 may stay near or against the lesser curvature of the stomach 10 as best shown in Fig. 11. In other words, the physician, while using laparoscopic observation through the stomach wall 11, may compare the position of light emitter 50 to a gastrointestinal tract landmark, such as pylorus 16, from an observation point outside of the stomach 10. After properly positioning gastric catheter 40, material may be suctioned from the gastrointestinal tract via side ports 45 of gastric catheter 40. Next, the gastric bougie 130, which was described earlier, may be slid over gastric catheter 40 and into the patient's stomach 10 in order to complete the gastric medical device assembly 120. The gastric bougie 130 may be advanced until its distal tip contact surface 34 comes in contact with the distal stop surface 44 of gastric catheter 40 as best shown in Fig. 1. This hard stop gives the physician tactile feedback to confirm proper placement of gastric bougie 130. Thus, in this example, the light emitter 50 is located at the distal end of gastric catheter 40 and the distal end of the gastric bougie 130 is positioned relative to light emitter 50, but this step can be done blindly by exploiting the contact between respective stop surfaces. In other words, the contact between the distal end of the gastric bougie 130 and distal stop surface 44 of the gastric catheter 40 can block movement of the gastric bougie 130 beyond the desired positioning in the patient's stomach 10. As discussed earlier, light emitter 50 may be a fiber optic light such that light is transmitted from an external light source through a fiber optic cable 23. Alternatively, the light emitter 50 may be an electrical light 59 that receives power from either an internal battery or an external power source as discussed earlier. After the gastric medical device 120 has been properly positioned in the patient's stomach 10 as shown in Fig. 13, one may apply further suction to collapse the stomach 10 around gastric bougie 130 utilizing side ports 135 in order to create an outline and identify locations for the staple line as shown with a dashed line throughout the story board. Identifying the outline may be further facilitated by activating light emitters 53 that are positioned in the stomach section of gastric bougie 130. Because of the translucency of the gastric bougie 130, this string of light emitters 53 can be observed laparoscopically from an observation point outside of the patient's stomach so that the physician may then to guide a stapler (not shown). In addition, the physician may utilize the outline provided by light emitters 53 to guide the staple/cutting procedure of the stomach to produce gastric sleeve 15 and separate the excised portion of the stomach 14, which may then be removed in a known manner from the patient.

The present description is for illustrative purposes only, and should not be construed to narrow the breadth of the present disclosure in any way. Thus, those skilled in the art will appreciate that various modification might be made to the presently disclosed embodiments without departing from the full and fair scope of the present disclosure. Other aspects, features and advantages will be apparent upon an examination of the attached drawings and appended claims.

## Claims

1. A gastric medical device assembly (20) comprising:
a gastric bougie (30) that defines at least one lumen (32);
a gastric catheter (40) with a distal segment (41) extending distally from the gastric bougie (30), a middle segment (42) slidably received in a first lumen of the at least one lumen (32), and a proximal segment extending proximally from the gastric bougie;
a light emitter (50) attached to a distal portion of at least one of the gastric bougie (30) and the gastric catheter (40), and having a luminosity sufficient to penetrate through, and be seen on an opposite side of, a stomach wall;
at least one of the gastric bougie (30) and the gastric catheter (40) define a plurality of side ports;
**characterized in that** the gastric catheter (40) includes a distal stop surface (44);
the gastric bougie (30) includes a tapered distal end (33) that terminates at a contact surface; and
the distal stop surface (44) blocks the gastric bougie (30) from being advanced past a position in contact with the distal stop surface (44) of the gastric catheter (40).

2. The gastric medical device assembly of any preceding claim including an external light source (70) luminously connected to the light emitter by a fiber optic cable (23).

3. The gastric medical device assembly of any preceding claim wherein the light emitter is one of a plurality of light emitters; and
a first portion of the light emitters being located inside the gastric bougie, and a remaining portion of the light emitters being positioned outside the gastric bougie.

4. The gastric medical device assembly of any preceding claim wherein the light emitter is one of a plurality of light emitters distributed in a pattern (56) along a length (57) of at least one of the gastric bougie and the gastric catheter.

5. The gastric medical device assembly of any preceding claim wherein the gastric catheter includes the plurality of light emitters distributed in the pattern (56) along the length (57) of the gastric catheter; and the gastric bougie (30) is translucent with respect to the light emitters

6. The gastric medical device assembly of any preceding claim wherein the gastric bougie defines a first plurality of side ports; and
the gastric catheter defines a second plurality of side ports.

7. The gastric medical device assembly of any preceding claim wherein the light emitter is attached to the distal portion of the gastric catheter and the distal stop surface (44) is located proximal to the light emitter.

8. The gastric medical device assembly of any preceding claim wherein the gastric catheter includes the plurality of light emitters distributed in the pattern (56) along the length (57) of the gastric catheter; and the gastric bougie is translucent with respect to the light emitters.

9. The gastric medical device assembly of any preceding claim wherein the gastric catheter (40) has a closed atraumatic distal tip (46) and defines a plurality of lumens;
the gastric catheter defines a plurality of side ports that are fluidly connected to a first lumen of the plurality of lumens; and
an energy transmission cable positioned in a second lumen of the plurality of lumens and being connected to the light emitter.

10. The gastric medical device assembly of claim 9 wherein the energy transmission cable includes an electrical circuit (25) and the light emitter includes an electrical light electrically connected to the electrical circuit.

11. The gastric medical device assembly of any preceding claim wherein the energy transmission cable includes a fiber optic cable (23).

## Patentansprüche

1. Gastrische medizinische Vorrichtungsanordnung (20), umfassend:
eine gastrische Bougie (30), die mindestens ein Lumen (32) definiert;
einen Magenkatheter (40) mit einem distalen Segment (41), das sich distal von der gastrischen Bougie (30) erstreckt, einem mittleren Segment (42), das gleitfähig in einem ersten Lumen des mindestens einen Lumens (32) aufgenommen wird, und einem proximalen Segment, das sich proximal von der gastrischen Bougie erstreckt;
einen Lichtemitter (50), der an einem distalen Anteil von mindestens einem von der gastrischen Bougie (30) und dem Magenkatheter (40) angebracht ist und eine ausreichende Leuchtkraft aufweist, um eine Magenwand zu durchdringen und auf einer entgegengesetzten Seite von dieser gesehen zu werden;
wobei mindestens eins von der gastrischen Bougie (30) und dem Magenkatheter (40) eine Vielzahl von seitlichen Anschlüssen definiert;
**dadurch gekennzeichnet, dass** der Magenkatheter (40) eine distale Anschlagfläche (44) einschließt;
die gastrische Bougie (30) ein sich verjüngendes distales Ende (33) einschließt, das an einer Kontaktfläche endet; und
die distale Anschlagfläche (44) verhindert, dass die gastrische Bougie (30) über eine Position in Kontakt mit der distalen Anschlagfläche (44) des Magenkatheters (40) hinaus vorgeschoben werden kann.

2. Gastrische medizinische Vorrichtungsanordnung nach einem der vorhergehenden Ansprüche, einschließlich einer externen Lichtquelle (70), die durch ein faseroptisches Kabel (23) in Leuchtverbindung mit dem Lichtemitter steht.

3. Gastrische medizinische Vorrichtungsanordnung nach einem der vorhergehenden Ansprüche, wobei der Lichtemitter einer von einer Vielzahl von Lichtemittern ist; und
ein erster Anteil der Lichtemitter sich im Inneren der gastrischen Bougie befindet, und ein verbleibender Anteil der Lichtemitter außerhalb der gastrischen Bougie positioniert ist.

4. Gastrische medizinische Vorrichtungsanordnung nach einem der vorhergehenden Ansprüche, wobei der Lichtemitter einer von einer Vielzahl von Lichtemittern ist, die in einem Muster (56) entlang einer Länge (57) von mindestens einem von der gastrischen Bougie und dem Magenkatheter verteilt sind.

5. Gastrische medizinische Vorrichtungsanordnung nach einem der vorhergehenden Ansprüche, wobei der Magenkatheter die Vielzahl von Lichtemittern einschließt, die in dem Muster (56) entlang der Länge (57) des Magenkatheters verteilt sind; und
die gastrische Bougie (30) in Hinsicht auf die Lichtemitter durchscheinend ist.

6. Gastrische medizinische Vorrichtungsanordnung nach einem der vorhergehenden Ansprüche, wobei die gastrische Bougie eine erste Vielzahl von seitlichen Anschlüssen definiert; und
der Magenkatheter eine zweite Vielzahl von seitlichen Anschlüssen definiert.

7. Gastrische medizinische Vorrichtungsanordnung nach einem der vorhergehenden Ansprüche, wobei der Lichtemitter an dem distalen Anteil des Magenkatheters angebracht ist und die distale Anschlagfläche (44) sich proximal des Lichtemitters befindet.

8. Gastrische medizinische Vorrichtungsanordnung nach einem der vorhergehenden Ansprüche, wobei der Magenkatheter die Vielzahl von Lichtemittern einschließt, die in dem Muster (56) entlang der Länge (57) des Magenkatheters verteilt sind; und
die gastrische Bougie in Hinsicht auf die Lichtemitter durchscheinend ist.

9. Gastrische medizinische Vorrichtungsanordnung nach einem der vorhergehenden Ansprüche, wobei der Magenkatheter (40) eine geschlossene atraumatische distale Spitze (46) aufweist und eine Vielzahl von Lumina definiert;
der Magenkatheter eine Vielzahl von seitlichen Anschlüssen definiert, die fließtechnisch mit einem ersten Lumen von der Vielzahl der Lumina verbunden sind; und
ein Energieübertragungskabel in einem zweiten Lumen von der Vielzahl der Lumina positioniert ist und mit dem Lichtemitter verbunden ist.

10. Gastrische medizinische Vorrichtungsanordnung nach Anspruch 9, wobei das Energieübertragungskabel einen Stromkreis (25) einschließt, und der Lichtemitter ein elektrisches Licht einschließt, das elektrisch mit dem Stromkreis verbunden ist.

11. Gastrische medizinische Vorrichtungsanordnung nach einem der vorhergehenden Ansprüche, wobei das Energieübertragungskabel ein faseroptisches Kabel (23) einschließt.

## Revendications

1. Ensemble dispositif médical gastrique (20) comprenant :
une bougie gastrique (30) qui définit au moins une lumière (32) ;
un cathéter gastrique (40) assorti d'un segment distal (41) s'étendant distalement depuis la bougie gastrique (30), d'un segment intermédiaire (42) reçu de manière coulissante dans une première lumière de l'au moins une lumière (32), et d'un segment proximal s'étendant proximalement depuis la bougie gastrique ;
un émetteur de lumière (50) fixé à une partie distale de la bougie gastrique (30) et/ou du cathéter gastrique (40), et ayant une luminosité suffisante pour pénétrer à travers une paroi stomacale et être vu sur un côté opposé de celle-ci ;
la bougie gastrique (30) et/ou le cathéter gastrique (40) définissent une pluralité d'orifices latéraux ;
**caractérisé en ce que** le cathéter gastrique (40) comprend une surface de butée distale (44) ;
la bougie gastrique (30) comprend une extrémité distale conique (33) qui se termine au niveau d'une surface de contact ; et
la surface de butée distale (44) empêche que la bougie gastrique (30) soit avancée au-delà d'une position en contact avec la surface de butée distale (44) du cathéter gastrique (40).

2. Ensemble dispositif médical gastrique selon l'une quelconque des revendications précédentes comprenant une source de lumière externe (70) connectée de façon lumineuse à l'émetteur de lumière par un câble optique (23).

3. Ensemble dispositif médical gastrique selon l'une quelconque des revendications précédentes dans lequel l'émetteur de lumière est un émetteur d'une pluralité d'émetteurs de lumière ; et
une première partie des émetteurs de lumière étant située à l'intérieur de la bougie gastrique, et une partie restante des émetteurs de lumière étant située à l'extérieur de la bougie gastrique.

4. Ensemble dispositif médical gastrique selon l'une quelconque des revendications précédentes dans lequel l'émetteur de lumière est un émetteur d'une pluralité d'émetteurs de lumière distribués dans un motif (56) sur une longueur (57) de la bougie gastrique et/ou du cathéter gastrique.

5. Ensemble dispositif médical gastrique selon l'une quelconque des revendications précédentes dans lequel le cathéter gastrique comprend la pluralité d'émetteurs de lumière distribués dans le motif (56) sur la longueur (57) du cathéter gastrique ; et
la bougie gastrique (30) est translucide par rapport aux émetteurs de lumière.

6. Ensemble dispositif médical gastrique selon l'une quelconque des revendications précédentes dans lequel la bougie gastrique définit une première pluralité d'orifices latéraux ; et
le cathéter gastrique définit une seconde pluralité d'orifices latéraux.

7. Ensemble dispositif médical gastrique selon l'une quelconque des revendications précédentes dans lequel l'émetteur de lumière est fixé à la partie distale du cathéter gastrique et la surface de butée distale (44) est située à proximité de l'émetteur de lumière.

8. Ensemble dispositif médical gastrique selon l'une quelconque des revendications précédentes dans lequel le cathéter gastrique comprend la pluralité d'émetteurs de lumière distribués dans le motif (56) sur la longueur (57) du cathéter gastrique ; et
la bougie gastrique est translucide par rapport aux émetteurs de lumière.

9. Ensemble dispositif médical gastrique selon l'une quelconque des revendications précédentes dans lequel le cathéter gastrique (40) a une pointe distale atraumatique fermée (46) et définit une pluralité de lumières ;
le cathéter gastrique définit une pluralité d'orifices latéraux qui sont en communication fluidique avec une première lumière de la pluralité de lumières ; et
un câble de transmission d'énergie positionné dans une seconde lumière de la pluralité de lumières et étant connecté à l'émetteur de lumière.

10. Ensemble dispositif médical gastrique selon la revendication 9 dans lequel le câble de transmission d'énergie comprend un circuit électrique (25) et l'émetteur de lumière comprend une lumière électrique connectée électriquement au circuit électrique.

11. Ensemble dispositif médical gastrique selon l'une quelconque des revendications précédentes dans lequel le câble de transmission d'énergie comprend un câble optique (23).
